# FASCICULE DE BREVET EUROPEEN

(11) **EP 2 440 552 B1**
(45) Date de publication et mention de la délivrance du brevet: **18.06.2014**
(21) Numéro de dépôt: 10727018.3
(22) Date de dépôt: 03.06.2010
(51) Int. Cl.: C07D 407/10, C07D 407/14, H01L 51/00

(54) **SEMI-CONDUCTEURS ORGANIQUES DE TYPE N INCORPORANT AU MOINS DEUX GROUPEMENTS 2-DICYANOMETHYLENE-3-CYANO-2,5-DIHYDROFURANE**
ORGANISCHE N-HALBLEITER MIT MINDESTENS ZWEI 2-DICYANOMETHYLEN-3-CYANO-2,5-DIHYDROFURAN-GRUPPEN
N-TYPE ORGANIC SEMICONDUCTORS INCLUDING AT LEAST TWO 2-DICYANOMETHYLENE-3-CYANO-2,5-DIHYDROFURAN GROUPS

(30) Priorité: 09.06.2009 FR 0902777
(43) Date de publication de la demande: 18.04.2012
(73) Titulaire: Commissariat à l'Énergie Atomique et aux Énergies Alternatives, 75015 Paris (FR)
(72) Inventeur: SUSPENE, Clément, F-19000 Tulle (FR); SIMONATO, Jean-Pierre, F-38360 Sassenage (FR)
(74) Mandataire: Noel, Chantal Odile
(86) Numéro de dépôt international: PCT/FR2010/000409
(87) Numéro de publication internationale: WO 2010/142864

(56) Documents cités:
- CN-C- 100 386 321
- ADVANCED MATERIALS (WEINHEIM, GERMANY) , 16(4), 356-360 CODEN: ADVMEW; ISSN: 0935-9648, 2004, XP002571609
- GONG ET AL: "New ''Y'' type nonlinear optical chromophores with good transparency and enhanced nonlinear optical effects" MATERIALS LETTERS, NORTH HOLLAND PUBLISHING COMPANY. AMSTERDAM, NL, vol. 61, no. 4-5, 10 janvier 2007 (2007-01-10), pages 1151-1153, XP005825617 ISSN: 0167-577X
- ZHENG, SHIJUN ET AL: "Two-Photon Absorption in Quadrupolar Bis(acceptor)-Terminated Chromophores with Electron-Rich Bis(heterocycle)vinylene Bridges" 2007, CHEMISTRY OF MATERIALS , 19(3), 432-442 CODEN: CMATEX; ISSN: 0897-4756 , XP002571610 page 433

## Description

L'invention concerne une molécule comprenant au moins deux groupements 2-dicyanométhylène-3-cyano-2,5-dihydrofurane ainsi que ses utilisations.

L'utilisation de dispositifs électroniques tels que les écrans d'ordinateur et de télévision, les cartes bancaires ou les téléphones portables nouvelle génération est omniprésente dans notre vie quotidienne.

Les transistors sont les constituants essentiels de ces dispositifs.

Ces transistors sont basés, depuis 1947, en grande majorité sur des semi-conducteurs inorganiques et notamment sur le silicium.

Cependant, des limitations technologiques associées à l'utilisation de ce dernier ont induit récemment le développement de transistors intégrant des semi-conducteurs organiques.

La facilité de mise en oeuvre de ces composés, qui sont des polymères ou des petites molécules conjuguées, leur confère en effet un potentiel intéressant dans le domaine de l'électronique grande surface à bas coût, bien que leur mobilité soit inférieure à celle du silicium cristallin.

Ces semi-conducteurs organiques peuvent également être employés pour de nouvelles applications telles que les moyens modernes d'identification ou pour l'affichage électronique.

Les semi-conducteurs organiques de type n mettent en jeu le déplacement des électrons dans les dispositifs électroniques.

Cependant à l'heure actuelle, les semi-conducteurs organiques de type n ne sont pas nombreux et appartiennent généralement à la famille des fullerènes.

En effet, la grande majorité des semi-conducteurs organiques existants, que ce soient des polymères ou des petites molécules conjuguées, sont des conducteurs de type p, c'est-à-dire qu'ils transportent plus facilement les "trous" que les électrons.

Or, il y a actuellement un intérêt croissant dans le développement de transistors de type n, notamment pour la fabrication de circuits complémentaires.

Ces matériaux de type n doivent posséder une mobilité élevée, une affinité électronique suffisamment grande pour assurer une injection effective des électrons dans l'orbitale la plus basse vacante (LUMO) des molécules et enfin présenter une faible détérioration de leurs propriétés électroniques dans le temps.

Afin de pallier le faible nombre de semi-conducteurs de type n disponibles, une stratégie a été envisagée qui consiste à inverser le caractère p en n des semi-conducteurs de type p connus.

Cette inversion a été décrite plusieurs fois dans la littérature et consiste à lier des groupements électro-attracteurs à ces semi-conducteurs de type p.

Ces groupements électro-attracteurs incluent notamment les atomes de fluor, les groupements fluorocarbonés de type -(CF₂)ₙ-CF₃, les groupements cyanos, les groupements malononitriles, les groupements anhydrides ou les groupements imides.

Avec l'introduction de ces groupements dans les matériaux semi-conducteurs de type p, les électrons sont "attirés" vers ces groupements, le "coeur" des molécules devenant électro-déficient et pouvant donc servir au transport des charges négatives injectées.

Les mobilités des dispositifs formés à partir de films cristallins de ces composés peuvent maintenant atteindre environ 1 cm².v⁻¹.s⁻¹ en géométrie grille haute-contact haut, ce qui justifie l'intérêt croissant pour ces matériaux malgré le fait que la géométrie grille haute-contact haut donne des mobilités très supérieures aux mobilités des dispositifs à géométrie grille basse-contact bas.

Parmi les exemples les plus significatifs de semi-conducteurs de type n préparés par cette méthode, peuvent être cités les pérylènes diimides comme le PDI-8CN₂, les naphtalènes diimides ou les quaterthiophènes fluorés comme le DFCO-4T (M. Mas-Torrent et C. Rovira Chem. Soc. Rev. 2008, 37, 827).

Un problème majeur réside dans le fait que l'accroissement du caractère n d'une molécule est en général concomitant avec la diminution de sa solubilité dans les solvants organiques.

En effet, lors de l'utilisation de groupements fluor par exemple, il arrive que la solubilité des composés obtenus soit si faible que le dépôt des semi-conducteurs par voie liquide se révèle être impossible.

La sublimation devient alors la seule possibilité de fabriquer des dispositifs incorporant des cristaux ou des films cristallins de semi-conducteurs de type n, cette voie étant d'ailleurs la plus employée aujourd'hui.

Le cas des naphtalènes diimides comportant des chaines alkyles latérales perfluorées peut être cité (K. C. See, C. Landis, A. Sarjeant and H. E. Katz Chem. Mater. 2008, 20, 3609).

De plus, peu de semi-conducteurs de type n actuels sont stables à l'air, c'est-à-dire que leurs propriétés électroniques se détériorent généralement de façon progressive dans le temps.

Les quelques exemples existants concernent des naphtalènes ou pérylènes diimides, des phtalocyanines de cuivre et des thiophènes comportant des chaines latérales perfluorées ou des groupements fluoro ou des groupements cyano sur les cycles aromatiques.

La disponibilité en semi-conducteurs de type n manipulables en solution et stables à l'air est donc très limitée de nos jours, le PDI-8CN₂ étant le composé le plus utilisé répondant à ces critères.

Dès lors, l'invention a pour but de fournir des molécules ayant des propriétés semi-conductrices de type n tout en ayant une bonne solubilité dans un solvant organique, et en étant stables à l'air, c'est-à-dire comprenant au moins deux groupements 2-dicyanométhylène-3-cyano-2,5-dihydrofurane liés à un groupement à liaisons pi conjuguées comprenant au moins un cycle aromatique à 5 ou 6 chaînons.

Mais avant tout, l'invention propose un procédé de fabrication d'un transistor caractérisé en ce qu'il comprend une étape d'utilisation d'une molécule de formule (I) suivante :

(GEA)ₙ-R-(DCDHF)ₘ Formule (I)

dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 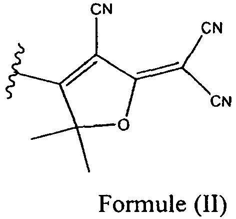
- m est un entier compris entre 2 et 10 inclus.

L'invention propose donc aussi un procédé de fabrication d'un transistor caractérisé en ce qu'il comprend une étape de dépôt, sur au moins une surface d'un substrat, d'une solution comprenant au moins une molécule de formule (I) suivante :

(GEA)ₙ-R-(DCDHF)ₘ Formule (I)

dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 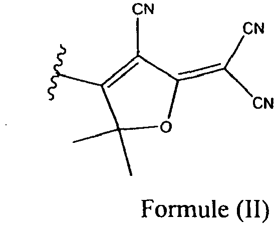
- m est un entier compris entre 2 et 10 inclus.

Dans le procédé de l'invention, de préférence, dans la molécule de formule (I), le au moins un cycle aromatique du groupement R est un cycle aromatique substitué par des groupements insaturés.

Toujours de préférence, la molécule de formule (I) a au moins un axe ou un plan de symétrie, avantageusement une symétrie C₂.

Encore de préférence, dans la molécule de formule (I) GEA est choisi parmi un atome de fluor, un groupe cyano, un groupe fluorocarboné, un groupe malononitrile, un groupe anhydride, un groupe imide, un groupe nitro, un groupe ammonium quaternaire, un groupe ester, un groupe amide, un groupe sulfonyle et les mélanges de ceux-ci.

Egalement de préférence, dans la molécule de formule (I), n est un entier compris entre 0 et 4 inclus.

Dans des modes de mise en oeuvre préférés, la molécule de formule (I) a l'une des formules 1 à 5 suivantes :

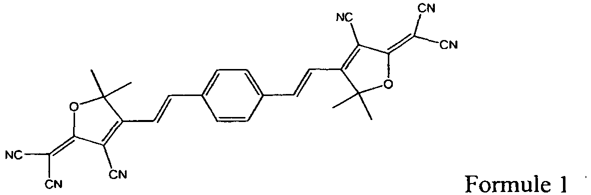

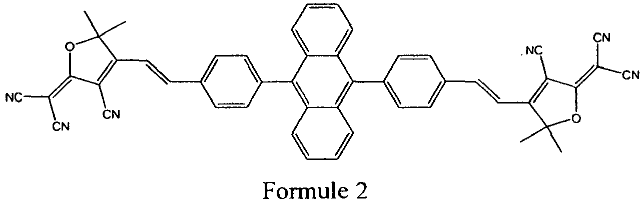

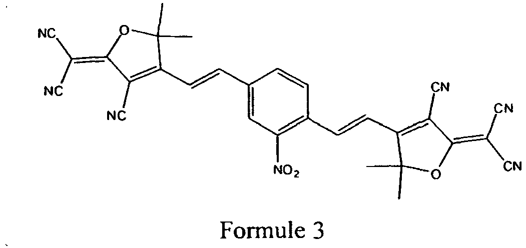

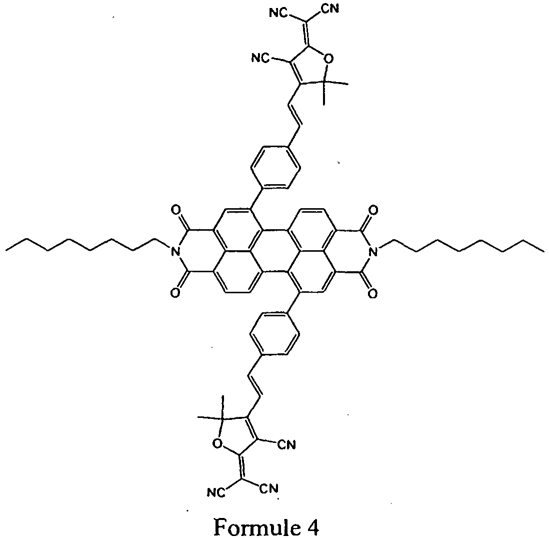

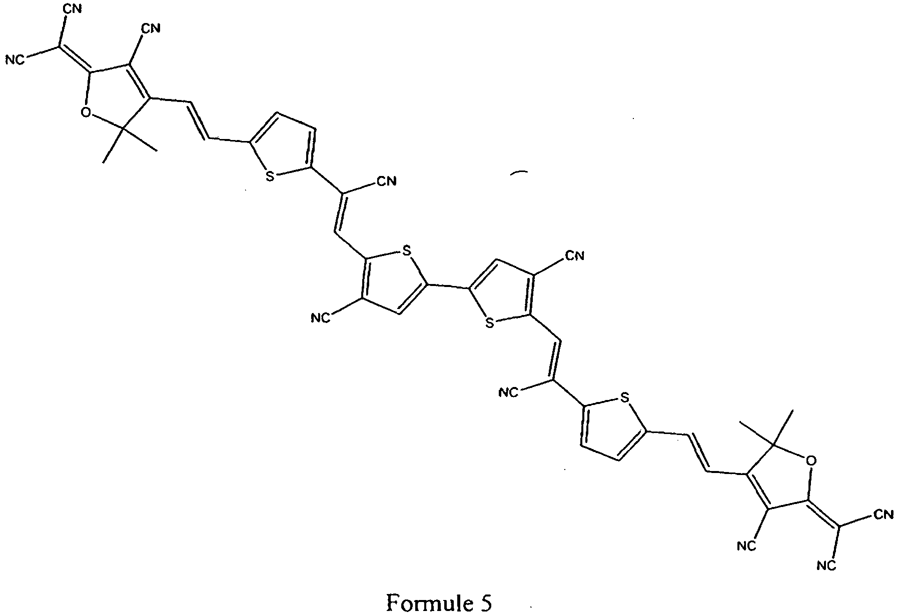

L'invention propose également une molécule caractérisée en ce qu'elle a la formule (I) suivante :

(GEA)ₙ-R-(DCDHF)ₘ Formule (I)

dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 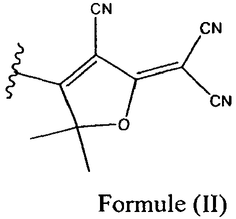
- m est un entier compris entre 2 et 10 inclus,
à la condition que lorsque n = 0, alors la molécule a la formule 1 ou 2 suivante :

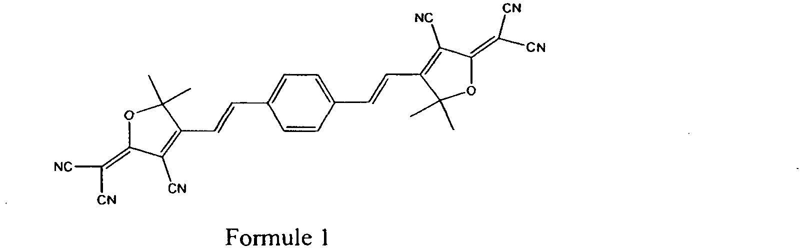

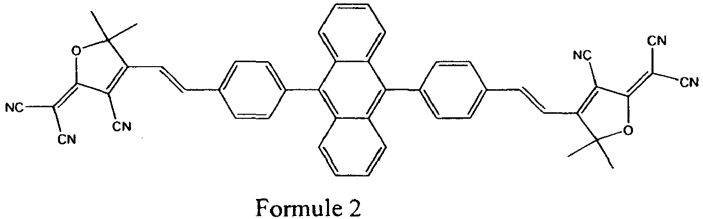

De préférence, cette molécule est choisie parmi les molécules de formules 1 à 5 suivantes :

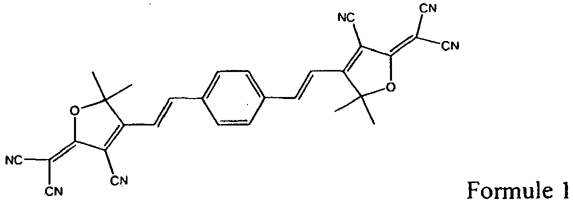

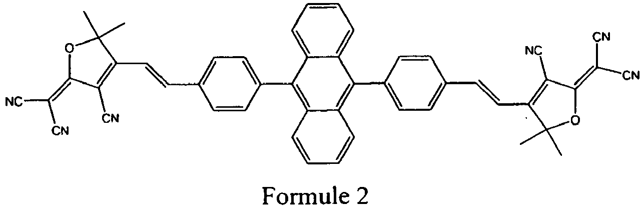

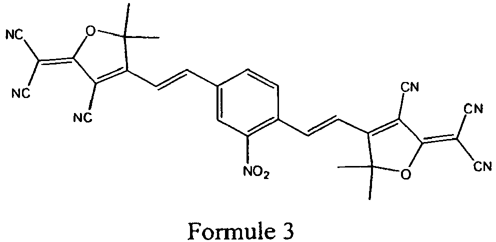

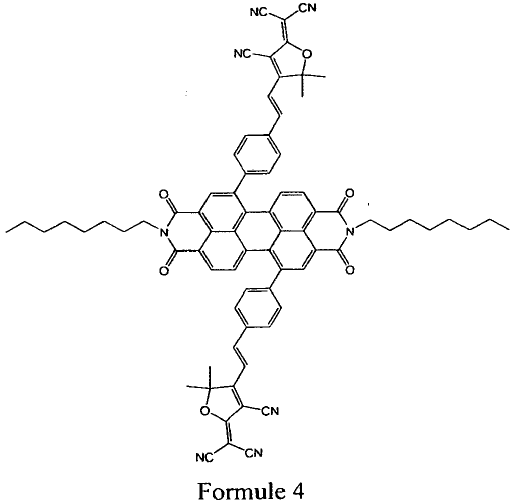

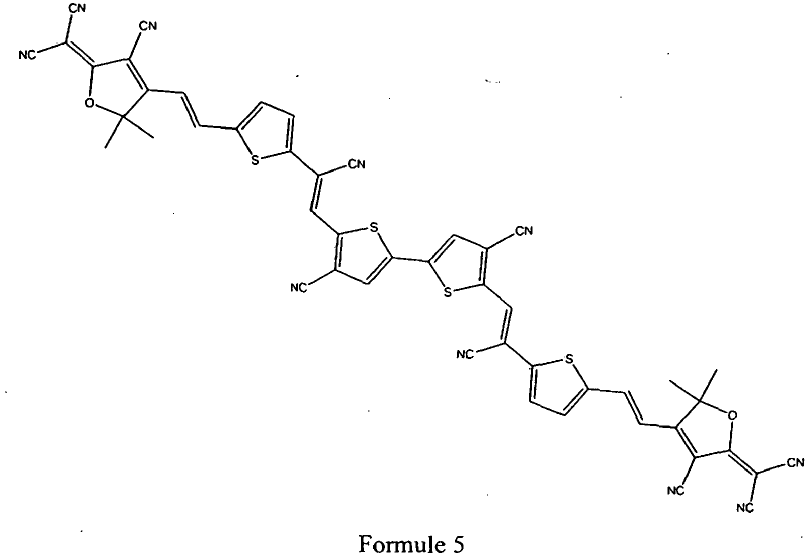

L'invention propose encore un transistor caractérisé en ce qu'il comprend au moins une couche comprenant au moins une molécule de formule (I) suivante :

(GEA)ₙ-R-(DCDHF)ₘ Formule (I)

dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 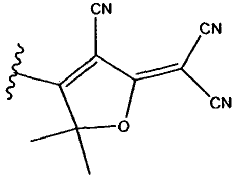 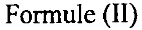
- m est un entier compris entre 2 et 10 inclus.

L'invention sera mieux comprise, et d'autres avantages et caractéristiques de celle-ci apparaîtront plus clairement à la lecture de la description explicative qui suit.

La molécule utilisée dans les procédés de fabrication de l'invention est une molécule ayant des propriétés semi-conductrices de type n, soluble dans un solvant organique et stable à l'air.

Cette molécule a deux caractéristiques essentielles : elle est constituée d'un groupement noté R à liaisons pi conjuguées comprenant au moins un cycle aromatique, et au moins deux groupements 2-dicyanométhylène-3-cyano-2,5-dihydrofurane sont liés directement ou indirectement à ce groupement R.

Le groupement aromatique peut de plus être lié, directement ou indirectement à des groupements électro-attracteurs, comme dans l'art antérieur.

Plus précisément, la molécule de l'invention a la formule (I) suivante :

(GEA)ₙ-R-(DCDHF)ₘ Formule (I)

Dans cette formule, R représente le groupement à liaisons pi conjuguées comprenant au moins un cycle aromatique ayant de 4, inclus, à 6, inclus, chaînons.

Cependant, de préférence, le groupement R comprend au moins un groupement phénylène, ou naphtalène, ou anthracène ou pérylène, ce cycle aromatique comprend optionnellement au moins un hétéroatome choisi parmi N, O, P, S, Si et Ge.

Il est également optionnellement substitué.

Lorsqu'il est substitué, les groupements GEA, lorsque présents, et DCDHF, sont liés à ces substituants.

De préférence, ces substituants sont des groupements insaturés, qui, dans un mode de réalisation particulièrement préféré, prolongent la conjugaison pi du ou des cycles aromatiques.

Dans la formule (I), DCDHF représente un groupement 2-dicyanométhylène-3-cyano-2,5-dihydrofurane de formule (II) suivante :

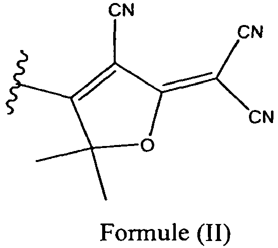

La molécule de formule (I) comprend au moins deux tels groupements DCDHF, ce qui signifie que m est un entier valant au moins 2.

De préférence, m est compris entre 2 et 10 inclus.

Egalement, dans la formule (I), GEA désigne un groupement électro-attracteur, tel que ceux connus dans l'art antérieur.

Ces groupements peuvent être présents ou non, ce qui entraîne que n est un entier supérieur ou égal à zéro.

De préférence, n est un entier compris entre 0 et 10 inclus, plus préférablement compris entre 0 et 4 inclus.

Le groupement GEA peut être, on l'a déjà dit, tout groupement électro-attracteur connu dans l'art antérieur tel qu'un atome de fluor ; un groupe cyano (-C=N) ; un groupe fluorocarboné de formule -(CF₂)ₓ-CF₃, avec x de préférence compris entre 0 et 17 ; un groupe malononitrile =CH-(CN)₂ ; un groupe anhydride O=C-O-C=O ; un groupe imide O=C-N-C=O, un groupe nitro -NO₂ ; un groupe ammonium quaternaire N⁺R₁R₂R₃ avec R₁, R₂ et R₃ représentant, indépendamment l'un de l'autre, un groupement alkyle en C₁ à C₅, de préférence un groupement méthyle, éthyle ou butyle, ou un atome d'hydrogène ; un groupe ester C(=O)-O-R₅, avec R₅ représentant de préférence un groupement choisi parmi les groupements hydrocarbonés éventuellement substitués, tels qu'un groupement méthyle ou isopropyle ; un groupe amide -C(=O)-N(R₇)-R₆, avec R₆ et R₇ représentant de préférence un groupement choisi parmi les groupements hydrocarbonés éventuellement substitués, tels qu'un groupement méthyle ou isopropyle, et un atome d'hydrogène ; ou encore un groupe sulfonyle S(=O)₂(R₈), avec R₈ représentant de préférence un groupement choisi parmi les groupements hydrocarbonés éventuellement substitués, tels qu'un groupement méthyle ou isopropyle, et un atome d'hydrogène.

De préférence, le groupement GEA est un groupement électro-attracteur comprenant une fonction cyano, imide, ou est un atome de fluor ou une chaîne fluorocarbonée.

De préférence, la molécule de formule (I) présente au moins un axe ou un plan de symétrie, avantageusement une symétrie C₂, c'est-à-dire que la molécule possède un axe de symétrie perpendiculaire à la chaîne de la molécule.

Autrement dit, une rotation de 180° autour de l'axe de symétrie C₂ donne la même molécule.

Des exemples préférés d'une molécule de formule (I) sont les molécules de formules 1, 2, 3, 4 et 5 suivantes :

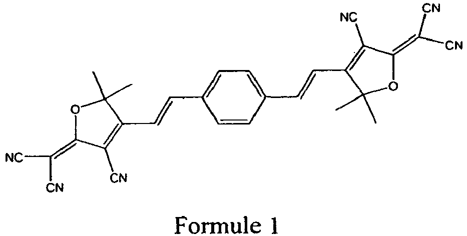

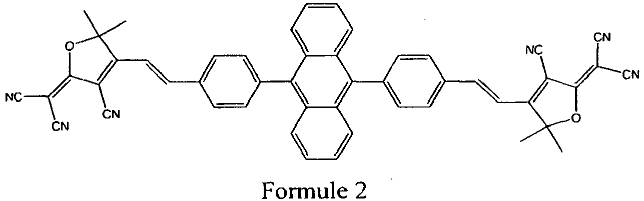

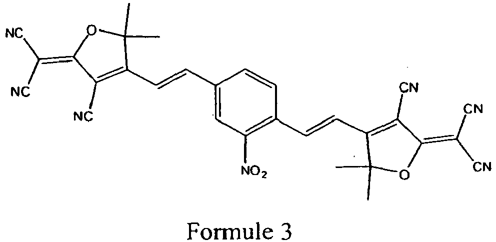

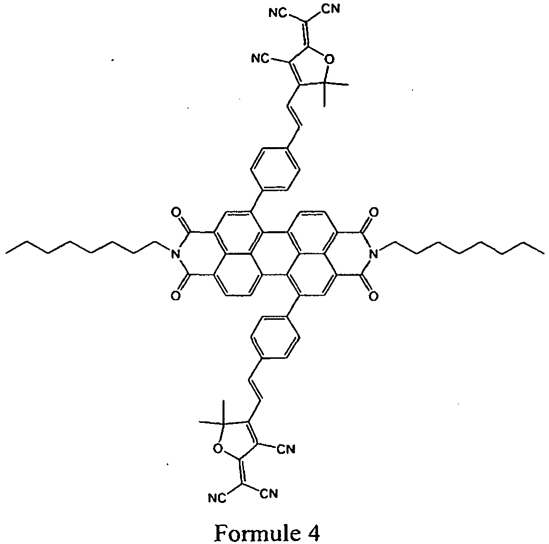

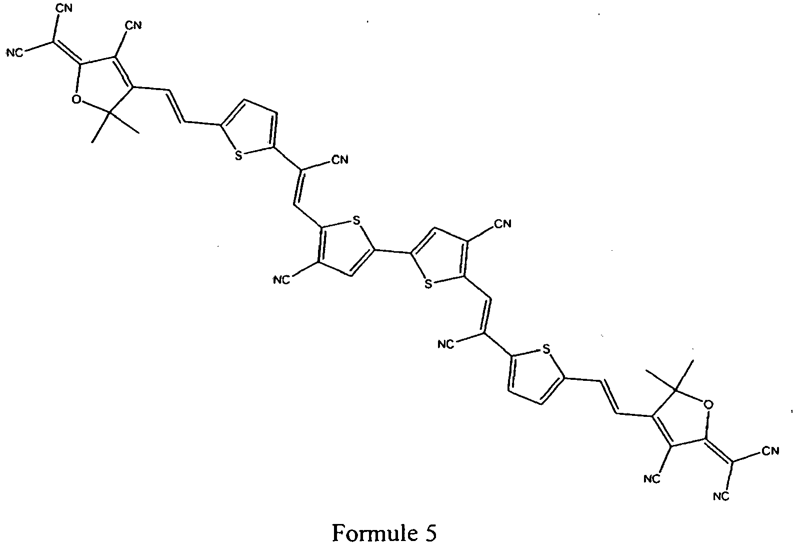

Les molécules de l'invention peuvent être dissoutes dans au moins un solvant organique.

A titre d'exemple non limitatif, ce solvant organique est l'acétone, le tétrahydrofurane, le diméthylformamide, l'acétonitrile, le benzonitrile, le chloroforme, l'*ortho*-dichlorobenzène ou le trifluorométhylbenzène.

Les molécules de l'invention ont des propriétés semi-conductrices de type n.

Elles peuvent donc être utilisées pour la fabrication d'un matériau semi-conducteur organique de type n et pour la formation d'un film en un matériau semi-conducteur organique de type n lorsqu'elles sont déposées sur au moins une surface d'un substrat.

Le dépôt de ces molécules peut être réalisé en voie liquide, c'est-à-dire en solution, par dépôt de gouttes, appelé "drop casting" en anglais, éventuellement à une température proche de la température d'ébullition du solvant choisi, ou par dépôt à la tournette, ou encore par jet d'encre.

La molécule de l'invention peut également être déposée par flexographie, par héliographie ou par formation de films Langmuir et Langmuir-Blodgett.

Mais elle peut également être déposée par sublimation sous vide.

En raison de leurs propriétés semi-conductrices de type n, les molécules de l'invention sont avantageusement utilisées dans un procédé de fabrication d'un transistor.

Plus précisément, un procédé de fabrication d'un transistor selon l'invention comprend une étape de dépôt, sur au moins une surface d'un substrat, d'une solution comprenant au moins une molécule selon l'invention.

Un autre procédé de fabrication d'un transistor selon l'invention comprend une étape d'utilisation d'une molécule de formule (I) telle que décrite ci-dessus.

L'invention concerne également un transistor qui comprend une couche en un matériau semi-conducteur organique de type n comprenant au moins une molécule de formule (I), plus préférablement ayant l'une des formules 1 à 5 ci-dessus.

L'invention propose également une molécule de formule (I) suivante :

(GEA)ₙ-R-(DCDHF)ₘ Formule (I)

dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 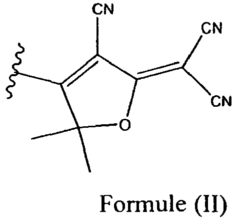
- m est un entier compris entre 2 et 10 inclus,
à la condition que lorsque n = 0, alors la molécule a la formule 1 ou 2 suivante :

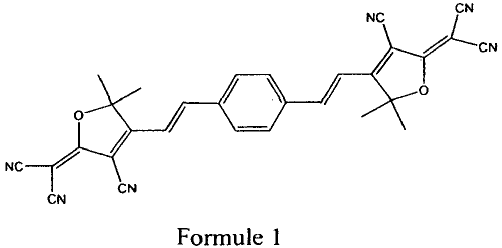

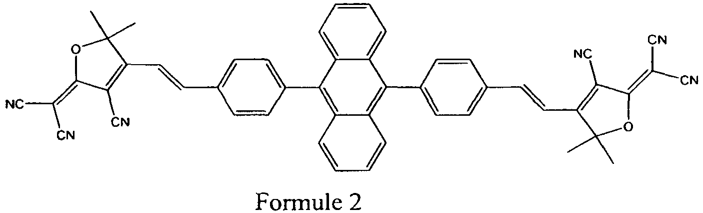

De préférence, cette molécule est choisie parmi les molécules de formules 1 à 5 suivantes :

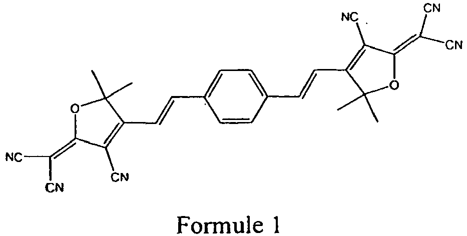

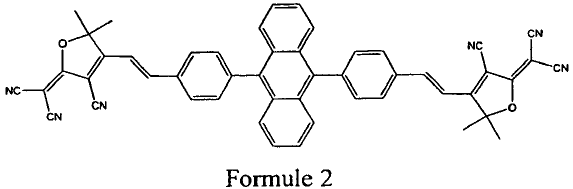

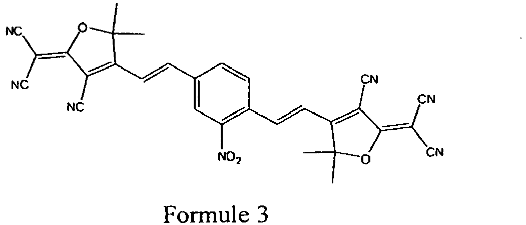

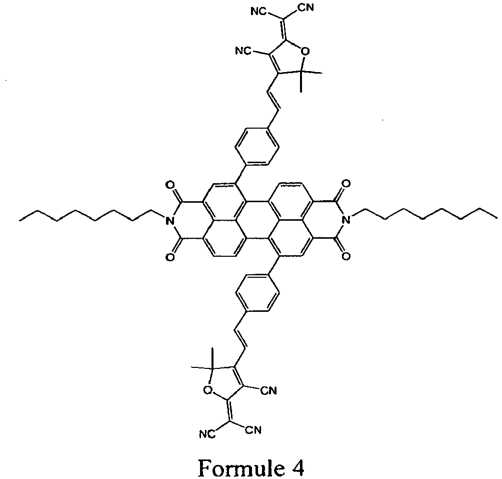

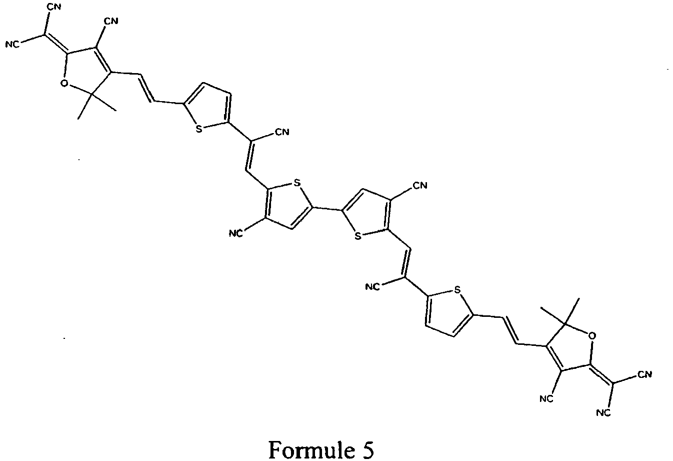

Afin de mieux faire comprendre l'invention, on va maintenant en décrire plusieurs modes de réalisation, qui sont donnés à titre purement illustratif et non limitatif.

### Exemple 1 : Synthèse d'une molécule selon l'invention.

Dans la molécule synthétisée à cet exemple, le groupement R est un groupement benzène.

La molécule de l'invention dans cet exemple ne contient pas de groupement électro-attracteur GEA (n = 0) mais possède deux groupements DCDHF liés au groupement benzène par un substituant insaturé (m = 2) :

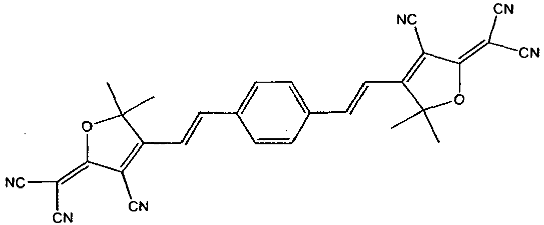

Dans ce cas, le DCDHF est d'abord synthétisé à part puis, grâce à l'acidité des protons du groupe méthyle situé en position α d'un des cyanos, il est introduit par réaction notamment de type Knoevenagel sur le téréphtaldialdéhyde. La double liaison issue de l'élimination d'une molécule d'eau lors de l'introduction du DCDHF permet de maintenir une conjugaison étendue sur toute la molécule, ce qui augmente la délocalisation des électrons.

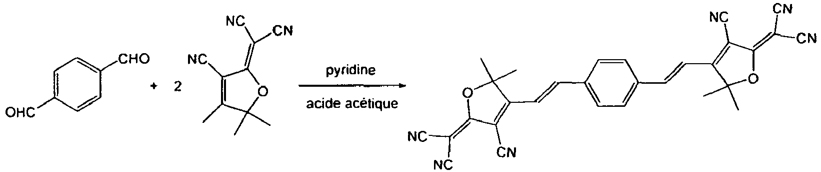

Le produit est obtenu sous la forme d'un solide rouge. Le dépôt par "drop casting" d'une solution de ce composé dans le diméthylformamide sur un transistor, quelle que soit sa géométrie, aboutit à la formation de cristaux.

### Exemple 2 : Synthèse d'une molécule selon l'invention.

La molécule de l'invention selon cet exemple comprend un groupement R qui est un anthracène et ne comprend pas de groupement GEA (n = 0).

La molécule possède deux groupements DCDHF liés au groupement aromatique par un substituant insaturé (m = 2) :

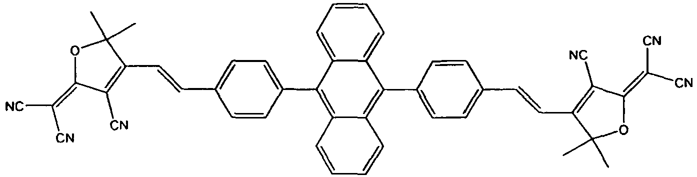

Ce composé est préparé par introduction de deux molécules de DCDHF par réaction de type Knoevenagel sur un dérivé bisaldéhydique de l'anthracène.

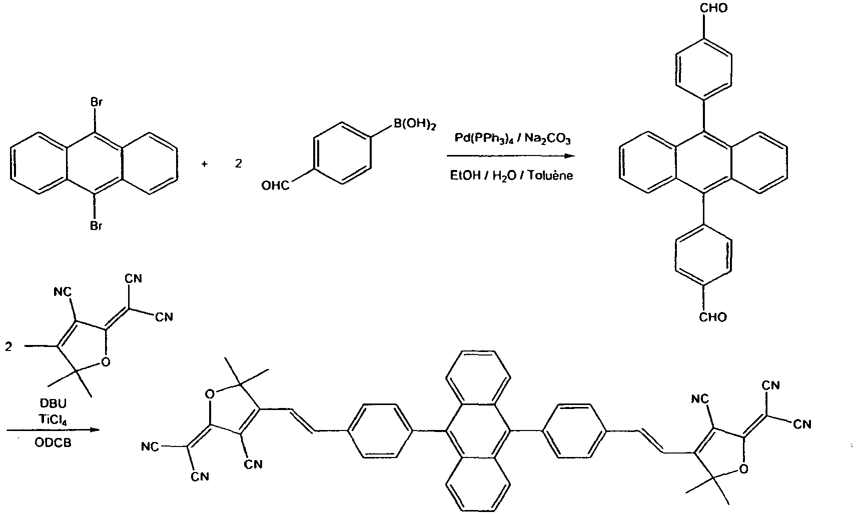

Le dérivé bisaldéhydique de l'anthracène est formé dans une première étape par couplage de Suzuki entre le 9,10-dibromoanthracène et l'acide 4-formylphénylboronique en présence d'une source de palladium et d'une base en milieu diphasique. Ensuite, celui-ci réagit avec deux équivalents de DCDHF dans l'*ortho-*dichlorobenzène en présence d'une base et d'un catalyseur, le tétrachlorure de titane. Le produit est obtenu sous la forme d'un solide rouge. Le dépôt par dépôt de gouttes d'une solution de ce composé dans le benzonitrile sur un transistor, quelle que soit sa géométrie, aboutit à la formation de cristaux.

### Exemple 3 : Synthèse d'une molécule selon l'invention.

Dans la molécule synthétisée à cet exemple, le groupement R est un groupement benzène.

La molécule de l'invention dans cet exemple contient un groupement électro-attracteur GEA de type nitro (n = 1) et possède deux groupements DCDHF liés au groupement benzène par un substituant insaturé (m = 2) :

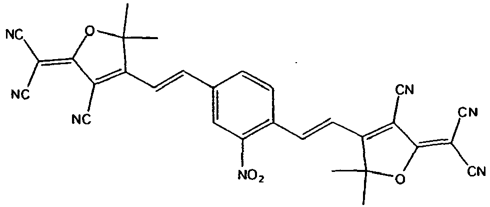

Dans ce cas, le DCDHF est d'abord synthétisé à part puis, grâce à l'acidité des protons du groupe méthyle situé en position α d'un des cyanos, il est introduit par réaction notamment de type Knoevenagel sur le 2-nitro-1,4-dibenzaldéhyde, lui-même obtenu par réduction du diacide correspondant. La double liaison issue de l'élimination d'une molécule d'eau lors de l'introduction du DCDHF permet de maintenir une conjugaison étendue sur toute la molécule, ce qui augmente la délocalisation des électrons.

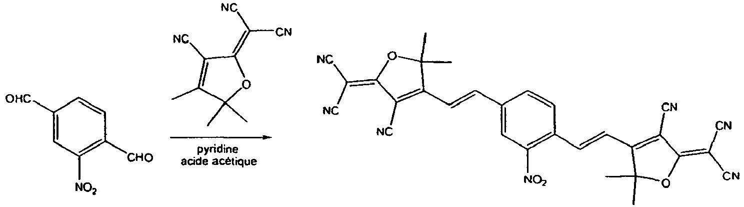

Le produit est obtenu sous la forme d'un solide brun. Le dépôt pour la réalisation des transistors est réalisé par "drop casting" d'une solution de ce composé dans le chloroforme.

### Exemple 4 : Synthèse d'une molécule selon l'invention.

Dans la molécule de l'invention selon cet exemple, le groupement R est un groupement pérylène lié à deux groupements électro-attracteurs GEA comprenant des fonctions imide (n = 2).

La molécule de cet exemple possède deux groupements DCDHF liés au groupement R par un substituant insaturé (m = 2) :

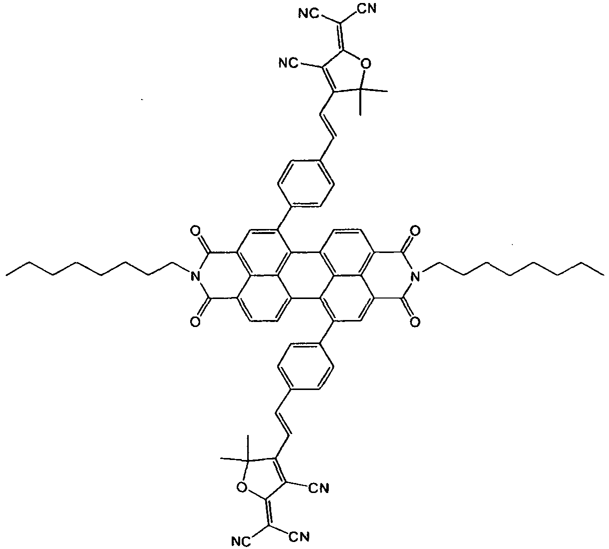

Ce composé est synthétisé à partir du pérylène tétracarboxylique dianhydride.

Le pérylène tétracarboxylique diimide est d'abord formé par réaction dans l'imidazole à 180 °C avec la *n*-octylamine puis une bromation de celui-ci est effectuée au reflux du dichlorométhane.

A ce stade, l'isomère 1,7 peut être isolé par chromatographie éventuellement suivie de recristallisations successives dans un mélange dichlorométhane/méthanol.

Enfin, par couplage de Suzuki avec l'acide 4-formylphénylboronique et réaction de Knoevenagel avec deux équivalents de DCDHF dans les conditions décrites dans l'exemple précédent, le produit est obtenu sous la forme d'un solide rouge.

Le dépôt par dépôt de gouttes d'une solution de ce composé dans le diméthylformamide sur un transistor, quelle que soit sa géométrie, aboutit à la formation de cristaux.

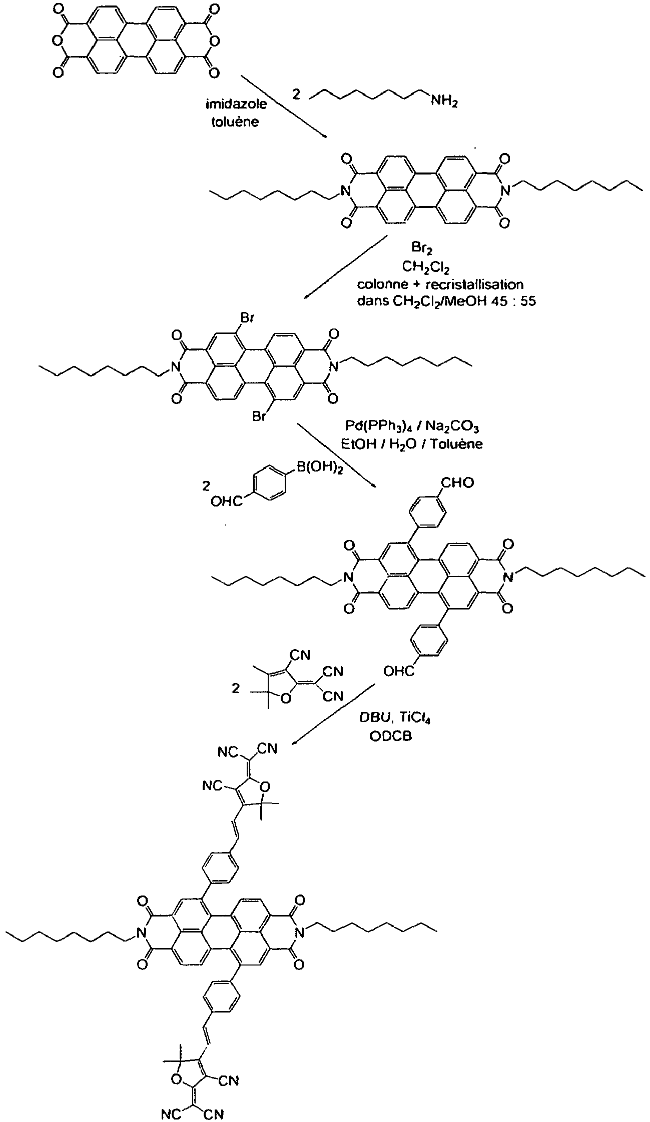

### Exemple 5 : Synthèse d'une molécule selon l'invention.

Dans la molécule synthétisée à cet exemple, le groupement R est un groupement bithiophène porteur de deux groupements thiophènevinylène.

La molécule de l'invention dans cet exemple contient quatre groupements électro-attracteurs GEA de type cyano (n = 4) et possède deux groupements DCDHF liés au groupement bithiophène porteur de deux groupements thiophènevinylène par un substituant insaturé (m = 2) :

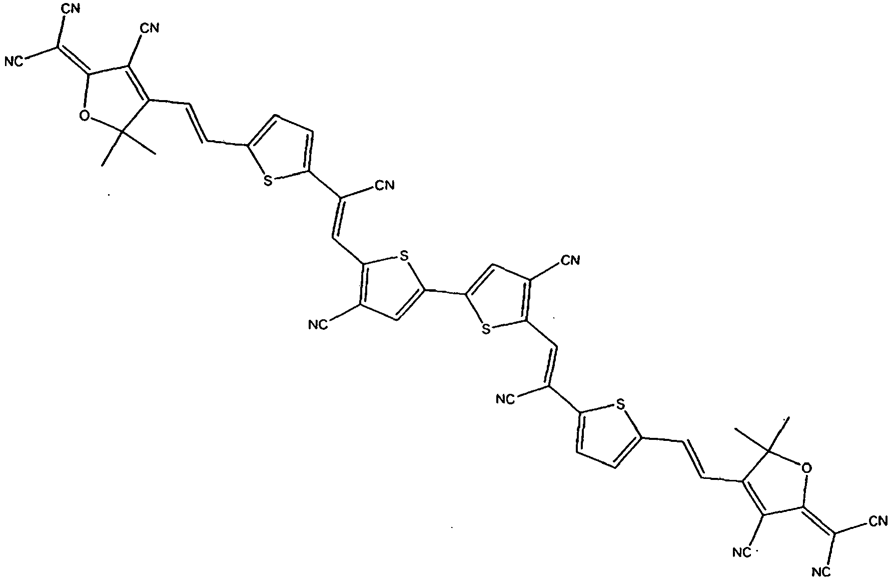

Dans ce cas, le 2-formyl-3-bromothiophène et le 2-formyl-5-acétonitrilothiophène sont tout d'abord synthétisés par réaction de Vilsmeier-Haak à partir respectivement du 3-bromothiophène et du 2-acétonitrilothiophène. Le DCDHF est ensuite introduit sur le 2-formyl-5-acétonitrilothiophène par réaction de type Knoevenagel.

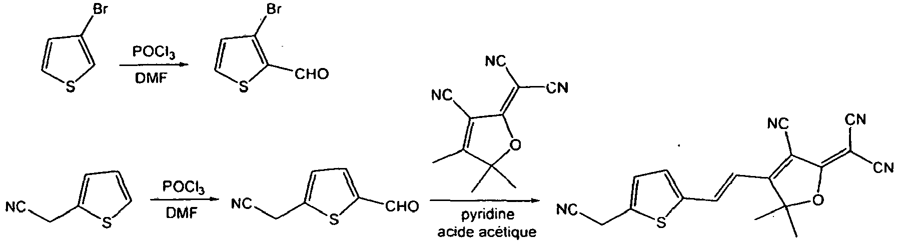

Le composé formé est additionné par réaction de type Knoevenagel sur le 2-formyl-3-bromothiophène puis l'atome de brome est substitué par un groupement cyano par réaction avec du dicyanure de zinc.

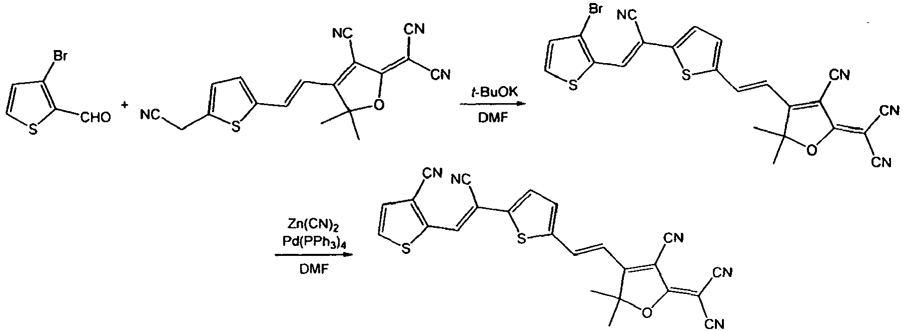

Le dérivé formé est ensuite mis en jeu dans deux réactions différentes. Dans l'une il est bromé avec du *N*-bromosuccinimide et dans l'autre un groupement triméthylétain est introduit.

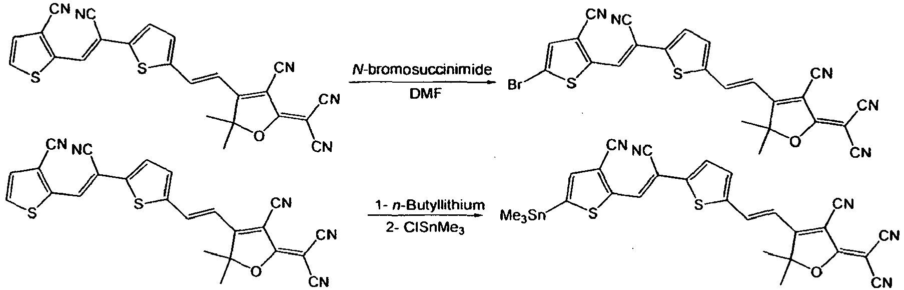

Une dernière étape impliquant une réaction de couplage entre les deux composés préparés en présence de palladium(II) permet d'aboutir au produit souhaité.

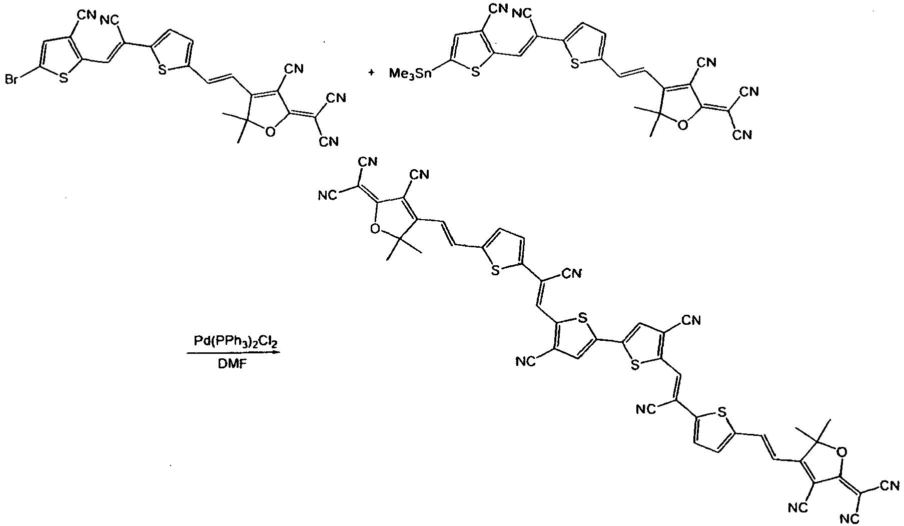

La conjugaison est étendue sur toute la molécule, ce qui augmente la délocalisation des électrons. Le produit est obtenu sous la forme d'un solide rouge. Le dépôt du semiconducteur organique est réalisé par "drop casting" d'une solution de ce composé dans le diméthylformamide.

### Exemple 6 : Fabrication de transistors à effet de champ avec les molécules des exemples 1 à 5 selon l'invention.

Différents transistors à effet de champ ont été fabriqués et notamment, à titre d'exemple, de la manière suivante (géométrie grille basse/contact bas, appelée "bottom-gate bottom-contact" en anglais) :
- sonication d'une puce (1 cm x 1 cm) comportant des transistors à motifs linéaires ou interdigités (largeur de canal W de 1000 à 10000 µm et longueur de canal L de 2 à 200 µm) dans l'acétone pendant 3 minutes puis rinçage à l'isopropanol et séchage à l'argon,
- traitement UV/ozone pendant 45 minutes,
- formation d'une couche auto-assemblée dans les canaux par trempage de la puce dans une solution d'octyltrichlorosilane dans le toluène (10⁻³ M) pendant 4 heures à 80 °C,
- dépôt du semi-conducteur de type n par une des méthodes précédemment évoquées,
- dégagement des électrodes des transistors au moyen d'un solvant organique appartenant à la liste présentée dans l'exposé de l'invention,
- recuit à 100 °C pendant 20 minutes,
- établissement des contacts électriques et notamment de l'électrode de grille basse,
- recuit à 100 °C pendant 5 minutes.

### Exemple 7 : Test des transistors obtenus à l'exemple 6.

### Les transistors à effet de champ obtenus à l'exemple 6 ont été testés.

Leurs valeurs de mobilité et de ratio Iₒₙ/I_{off}, déterminées par caractérisation électrique à l'air sont les suivantes :
- semi-conducteur de type n de l'exemple 1 : 3x10⁻⁵ cm².V⁻¹.s⁻¹ et 2x10²,
- semi-conducteur de type n de l'exemple 2 : 3x10⁻⁴ cm².V⁻¹.s⁻¹ et 3x10³,
- semi-conducteur de type n de l'exemple 3 : 1x10⁻⁴ cm².V⁻¹.s⁻¹ et 8x10²,
- semi-conducteur de type n de l'exemple 4 : 6x10⁻³ cm².V⁻¹.s⁻¹ et 8x10⁴,
- semi-conducteur de type n de l'exemple 5 : 8x10⁻⁴ cm².V⁻¹.s⁻¹ et 9x10².

Les exemples 6 et 7 montrent que les molécules de l'invention permettent d'obtenir des matériaux semi-conducteurs de type n ayant de bonnes propriétés de conduction et qui sont manipulables en solution et à l'air.

## Revendications

1. Procédé de fabrication d'un transistor **caractérisé en ce qu'**il comprend une étape d'utilisation d'une molécule de formule (I) suivante :
(GEA)ₙ-R-(DCDFH)ₘ Formule (I)
dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 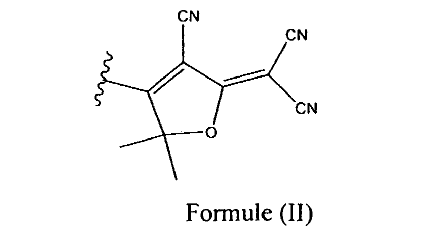
- m est un entier compris entre 2 et 10 inclus.

2. Procédé de fabrication d'un transistor **caractérisé en ce qu'**il comprend une étape de dépôt, sur au moins une surface d'un substrat, d'une solution comprenant au moins une molécule de formule (I) suivante :
(GEA)ₙ-R-(DCDHF)ₘ Formule (I)
dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 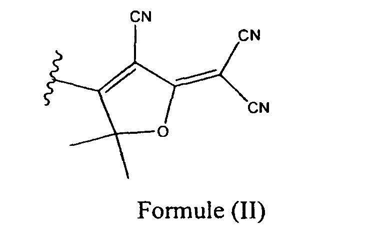
- m est un entier compris entre 2 et 10 inclus.

3. Procédé de fabrication d'un transistor selon la revendication 1 ou 2 **caractérisé en ce que**, dans la molécule de formule (I), le au moins un cycle aromatique du groupement R est un cycle aromatique substitué par des groupements insaturés.

4. Procédé de fabrication d'un transistor selon la revendication 1 ou 2 **caractérisé en ce que** le composé de formule (I) a au moins un axe ou un plan de symétrie, avantageusement une symétrie C₂.

5. Procédé selon l'une quelconque des revendications précédentes **caractérisé en ce que**, dans le composé de formule (I), GEA est choisi parmi un atome de fluor, un groupe cyano, un groupe fluorocarboné, un groupe malononitrile, un groupe anhydride, un groupe imide, un groupe nitro, un groupe ammonium quaternaire, un groupe ester, un groupe amide, un groupe sulfonyle et les mélanges de ceux-ci.

6. Procédé selon l'une quelconque des revendications précédentes **caractérisé en ce que**, dans la molécule de formule (I), n est un entier compris entre 0 et 4 inclus.

7. Procédé selon l'une quelconque des revendications précédentes **caractérisé en ce que** la molécule de formule (I) a l'une des formules suivantes :

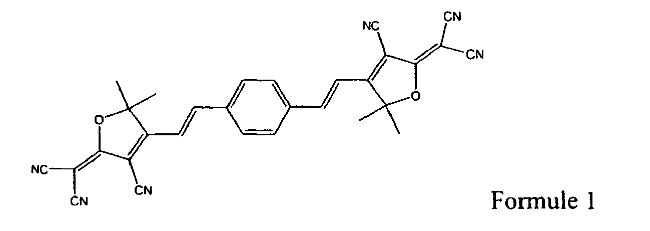

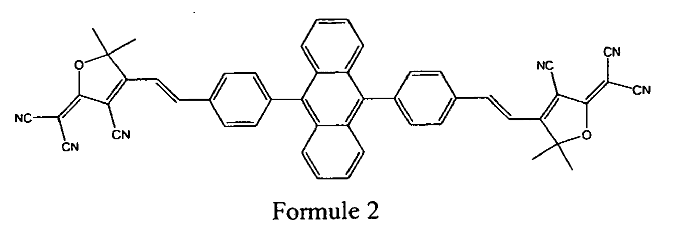

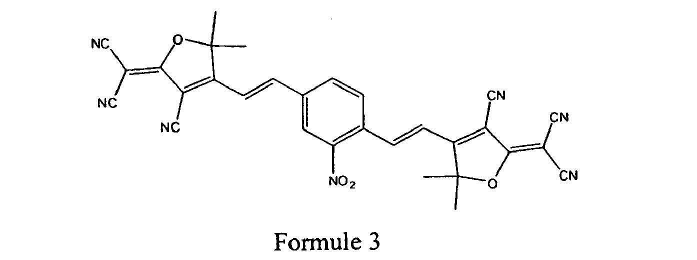

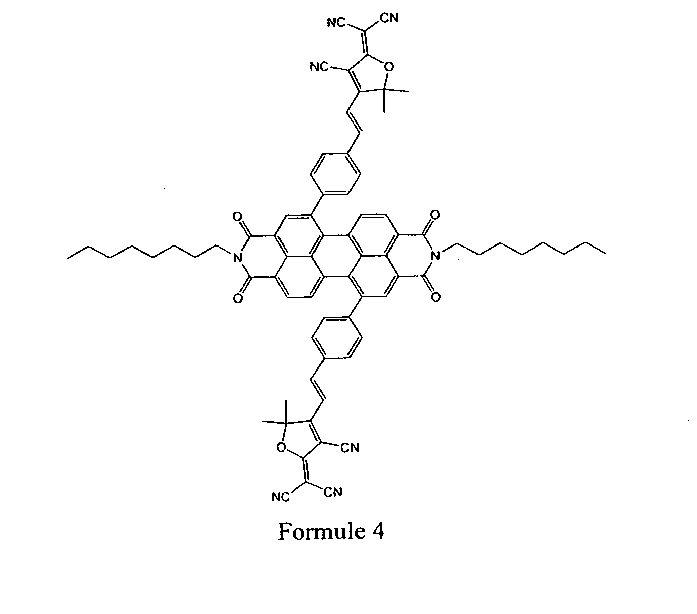

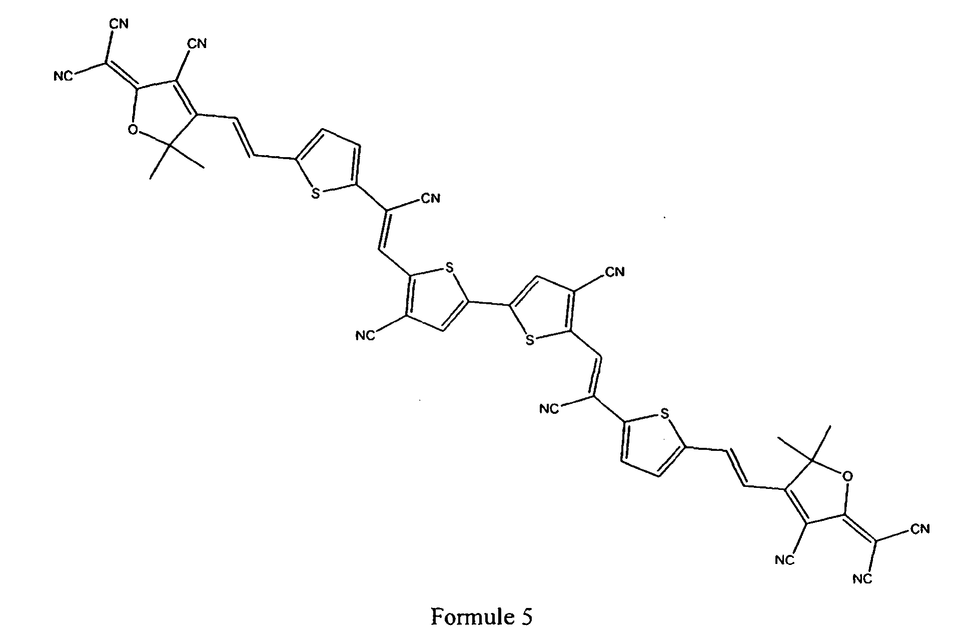

8. Molécule **caractérisée en ce qu'**elle a la formule (I) suivante :
(GEA)ₙ-R-(DCDHF)ₘ Formule (I)
dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 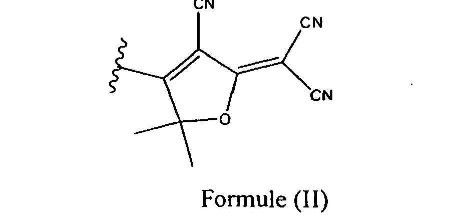
- m est un entier compris entre 2 et 10 inclus, à la condition que lorsque n = 0, alors la molécule a la formule 1 ou 2 suivante :

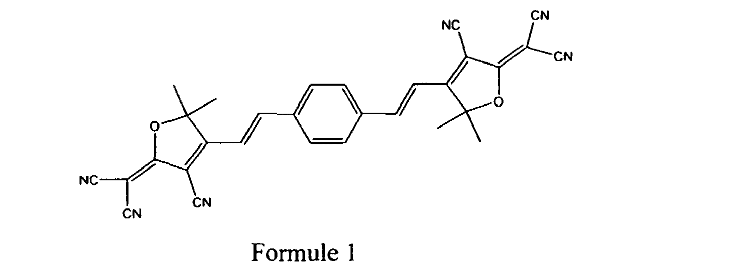

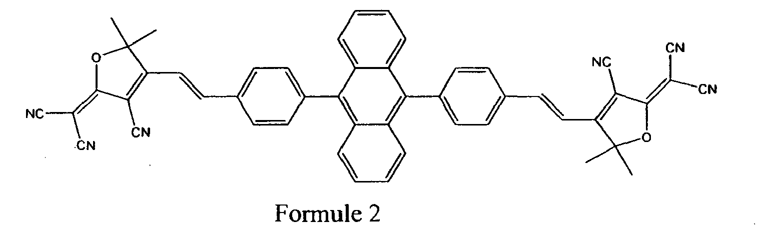

9. Molécule selon la revendication 8, **caractérisée en ce qu'**elle est choisie parmi les molécules de formules 1 à 5 suivantes :

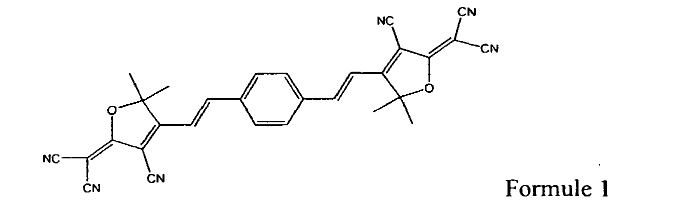

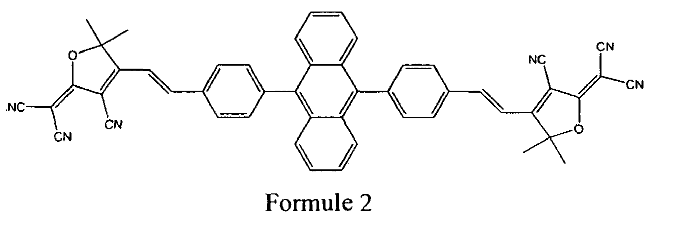

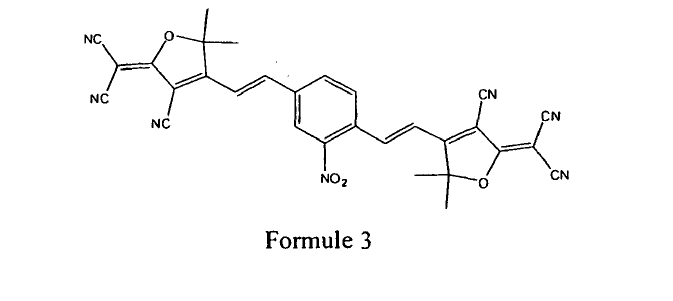

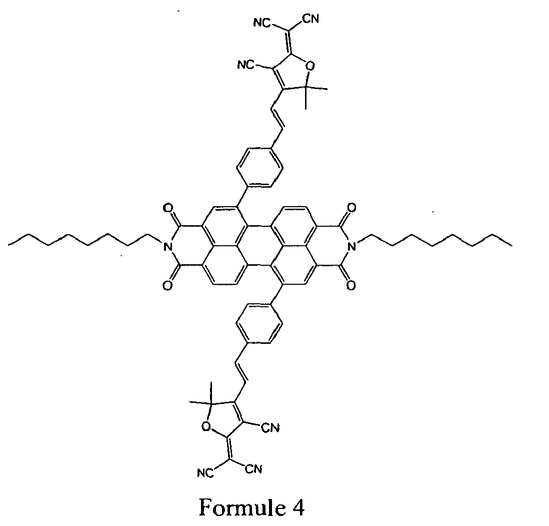

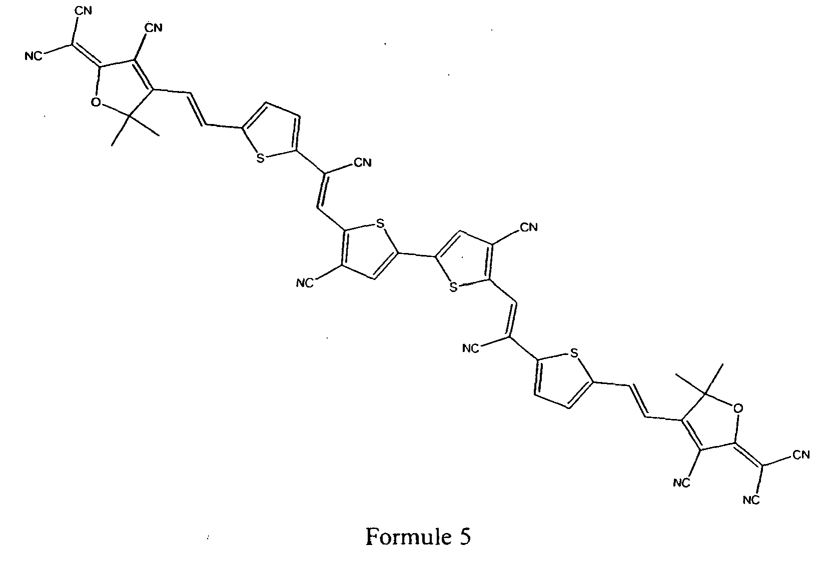

10. Transistor **caractérisé en ce qu'**il comprend au moins une couche comprenant au moins une molécule de formule (I) suivante :
(GEA)ₙ-R-(DCDHF)ₘ Formule (I)
dans laquelle :
- GEA désigne un groupement électro-attracteur,
- n est un entier positif compris entre zéro et 10 inclus,
- R est un groupement à liaisons pi conjuguées comprenant au moins un cycle aromatique ayant de 4 à 6 chaînons inclus, ce cycle aromatique comprenant optionnellement au moins un hétéroatome choisi parmi N, O, P, S, Si et Ge, et étant optionnellement substitué,
- DCDHF représente un groupement 2-dicyanométhylène-3-cyano-2,5-dihydrofurane de formule (II) suivante : 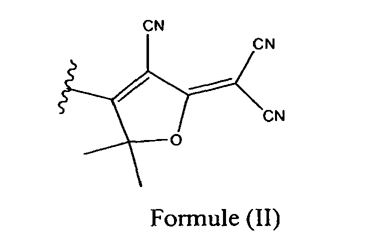
- m est un entier compris entre 2 et 10 inclus.

## Patentansprüche

1. Verfahren zur Herstellung eines Transistors, **dadurch gekennzeichnet, dass** es eine Stufe der Verwendung eines Moleküls der folgenden Formel (I) umfasst:
(GEA)ₙ-R**-**(DCDHF)ₘ Formel (I)
in der:
- GEA eine elektronenanziehende Gruppe bezeichnet,
- n eine positive ganze Zahl zwischen 0 und 10, einschließlich, ist,
- R eine Gruppe mit konjugierten pi-Bindungen ist, die wenigstens einen aromatischen Ring mit 4 bis 6 Kettengliedern, einschließlich, umfasst, wobei dieser aromatische Ring gegebenenfalls wenigstens ein Heteroatom, ausgewählt aus N, O, P, S, Si und Ge, umfasst und gegebenenfalls substituiert ist,
- DCDHF eine 2-Dicyanomethylen-3-cyano-2,5-dihydrofuran-Gruppe der folgenden Formel (II) darstellt: 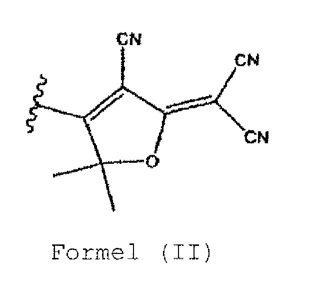
- m eine ganze Zahl zwischen 2 und 10, einschließlich, ist.

2. Verfahren zur Herstellung eines Transistors, **dadurch gekennzeichnet, dass** es eine Stufe der Abscheidung, auf wenigstens einer Oberfläche eines Substrates, einer Lösung umfasst, die wenigstens ein Molekül der folgenden Formel (I) umfasst:
(GEA)ₙ-R-(DCDHF)ₘ Formel (I)
in der:
- GEA eine elektronenanziehende Gruppe bezeichnet,
- n eine positive ganze Zahl zwischen 0 und 10, einschließlich, ist,
- R eine Gruppe mit konjugierten pi-Bindungen ist, die wenigstens einen aromatischen Ring mit 4 bis 6 Kettengliedern, einschließlich, umfasst, wobei dieser aromatische Ring gegebenenfalls wenigstens ein Heteroatom, ausgewählt aus N, O, P, S, Si und Ge, umfasst und gegebenenfalls substituiert ist,
- DCDHF eine 2-Dicyanomethylen-3-cyano-2,5-dihydrofuran-Gruppe der folgenden Formel (II) darstellt: 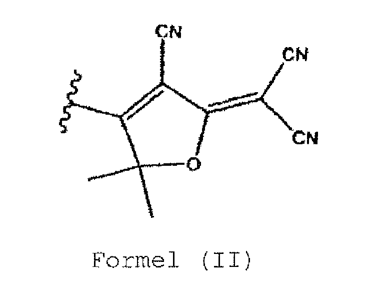
- m eine ganze Zahl zwischen 2 und 10, einschließlich, ist.

3. Verfahren zur Herstellung eines Transistors gemäß Anspruch 1 oder 2, **dadurch gekennzeichnet, dass** in dem Molekül der Formel (I) der wenigstens eine aromatische Ring der Gruppe R ein aromatischer Ring ist, der mit ungesättigten Gruppen substituiert ist.

4. Verfahren zur Herstellung eines Transistors gemäß Anspruch 1 oder 2, **dadurch gekennzeichnet, dass** die Verbindung der Formel (I) wenigstens eine Symmetrieachse oder -ebene, vorzugsweise eine C₂-Symmetrie, hat.

5. Verfahren gemäß einem der vorangehenden Ansprüche, **dadurch gekennzeichnet, dass** in der Verbindung der Formel (I) GEA aus einem Fluoratom, einer Cyanogruppe, einer Fluorkohlenstoffgruppe, einer Malononitrilgruppe, einer Anhydridgruppe, einer Imidgruppe, einer Nitrogruppe, einer quaternären Ammoniumgruppe, einer Estergruppe, einer Amidgruppe, einer Sulfonylgruppe und Gemischen dieser ausgewählt ist.

6. Verfahren gemäß einem der vorangehenden Ansprüche, **dadurch gekennzeichnet, dass** in dem Molekül der Formel (I) n eine ganze Zahl zwischen 0 und 4, einschließlich, ist.

7. Verfahren gemäß einem der vorangehenden Ansprüche, **dadurch gekennzeichnet, dass** das Molekül der Formel (I) eine der folgenden Formeln hat:

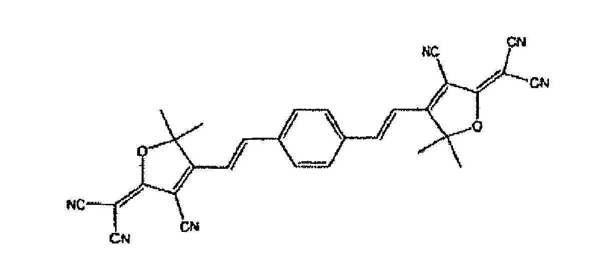

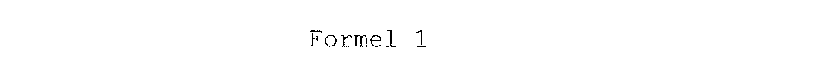

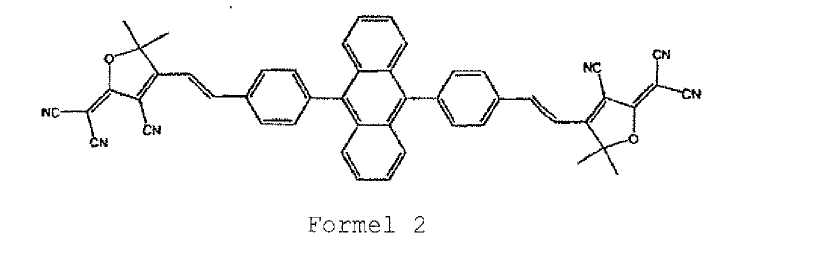

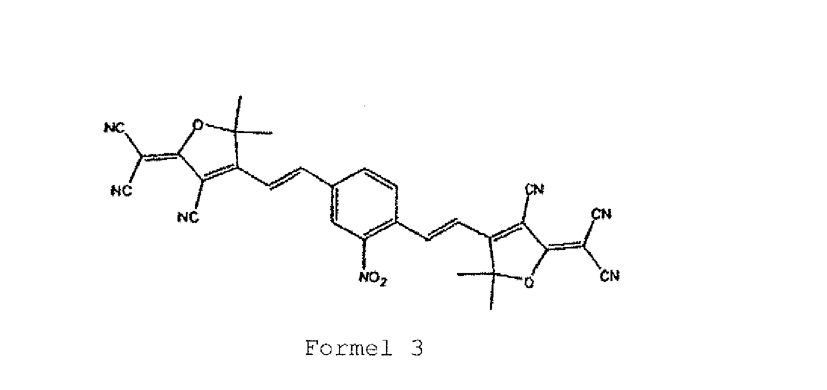

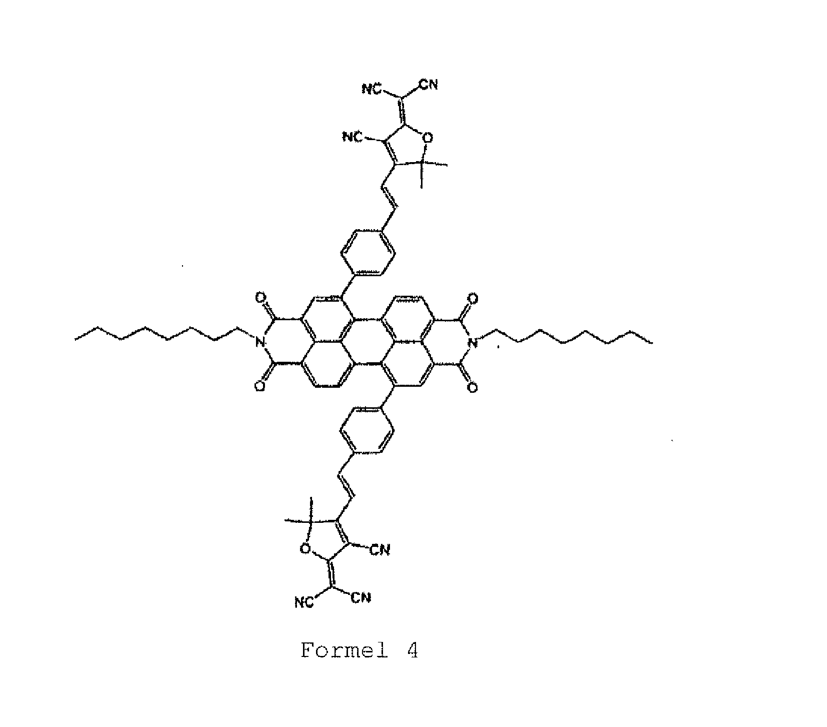

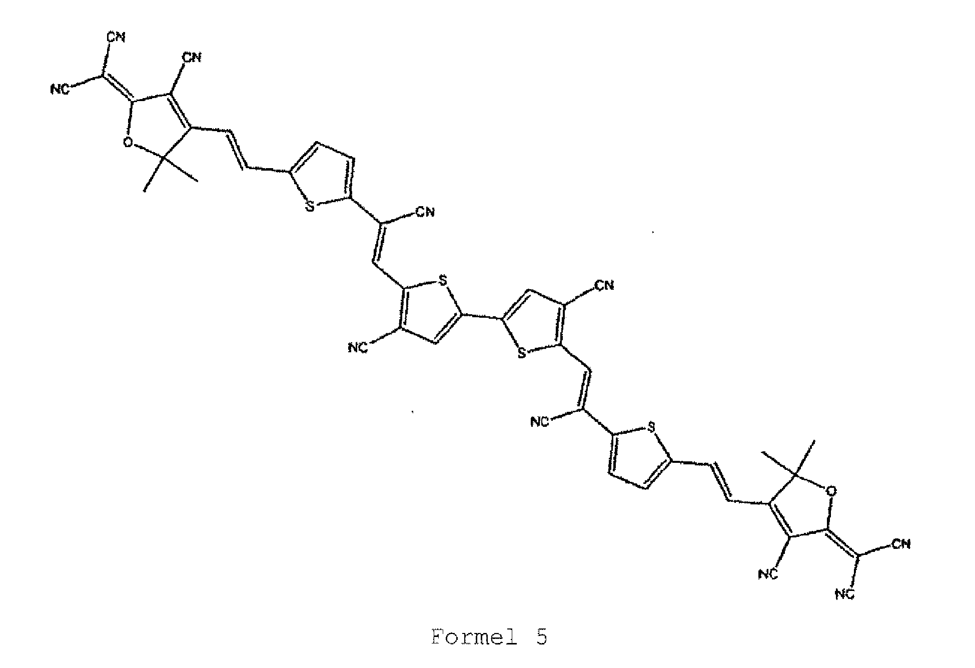

8. Molekül, **dadurch gekennzeichnet, dass** es die folgende Formel (I) hat:
(GEA)ₙ-R-(DCDHF)ₘ Formel (I)
in der :
- GEA eine elektronenanziehende Gruppe bezeichnet,
- n eine positive ganze Zahl zwischen 0 und 10, einschließlich, ist,
- R eine Gruppe mit konjugierten pi-Bindungen ist, die wenigstens einen aromatischen Ring mit 4 bis 6 Kettengliedern, einschließlich, umfasst, wobei dieser aromatische Ring gegebenenfalls wenigstens ein Heteroatom, ausgewählt aus N, 0, P, S, Si und Ge, umfasst und gegebenenfalls substituiert ist,
- DCDHF eine 2-Dicyanomethylen-3-cyano-2,5-dihydrofuran-Gruppe der folgenden Formel (II) darstellt: 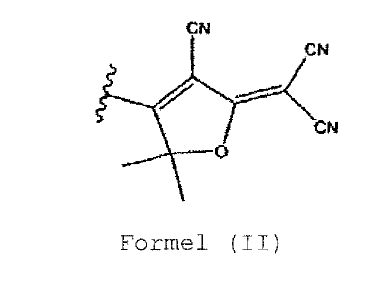
- m eine ganze Zahl zwischen 2 und 10, einschließlich, ist,
mit der Maßgabe, dass, wenn n = 0, dann das Molekül die folgende Formel 1 oder 2 hat:

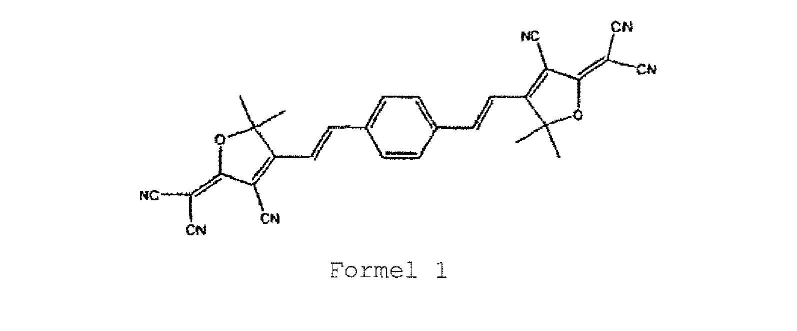

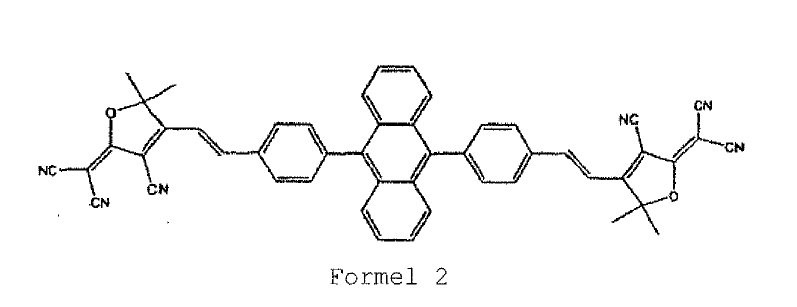

9. Molekül gemäß Anspruch 8, **dadurch gekennzeichnet, dass** es aus den Molekülen der folgenden Formeln 1 bis 5 ausgewählt ist:

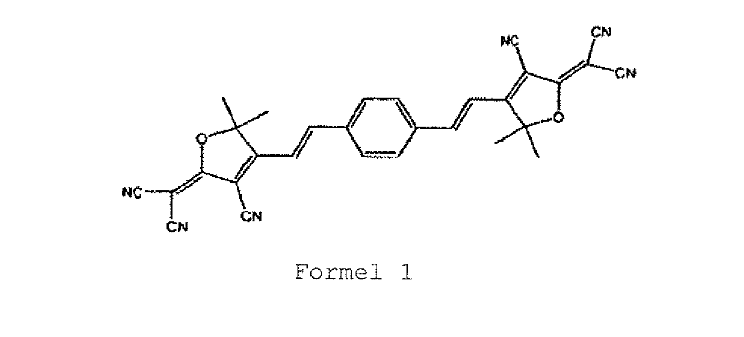

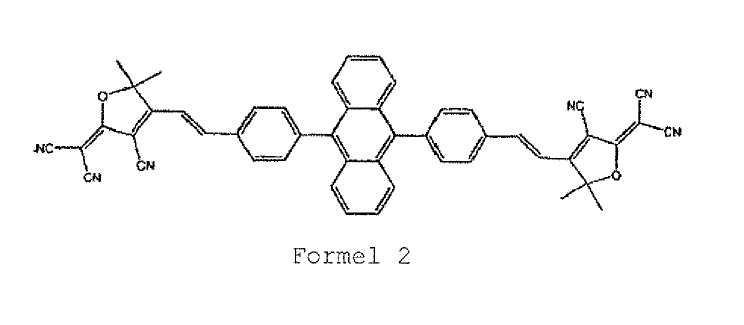

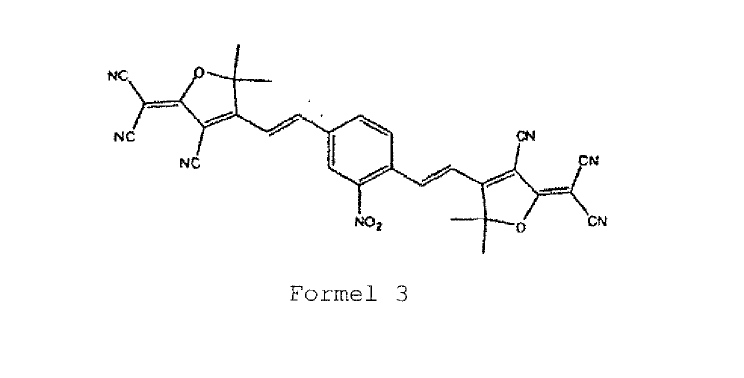

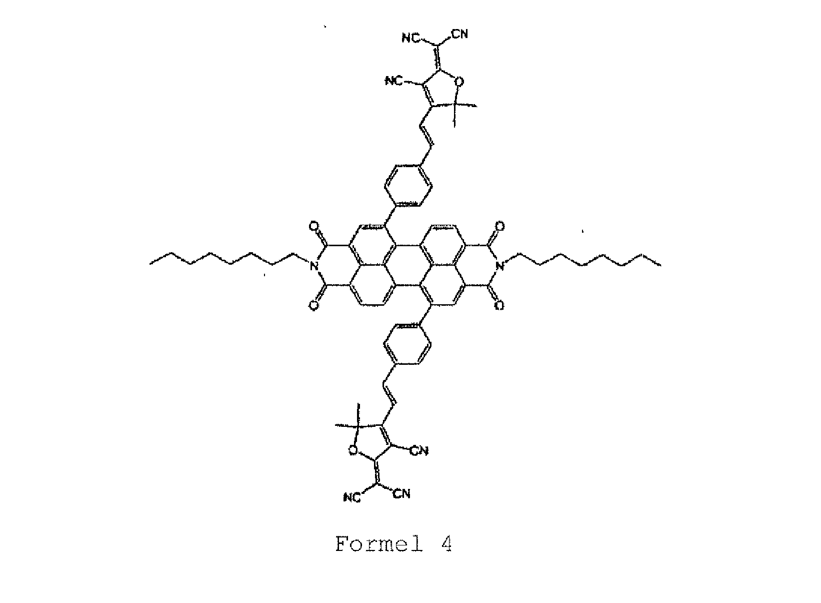

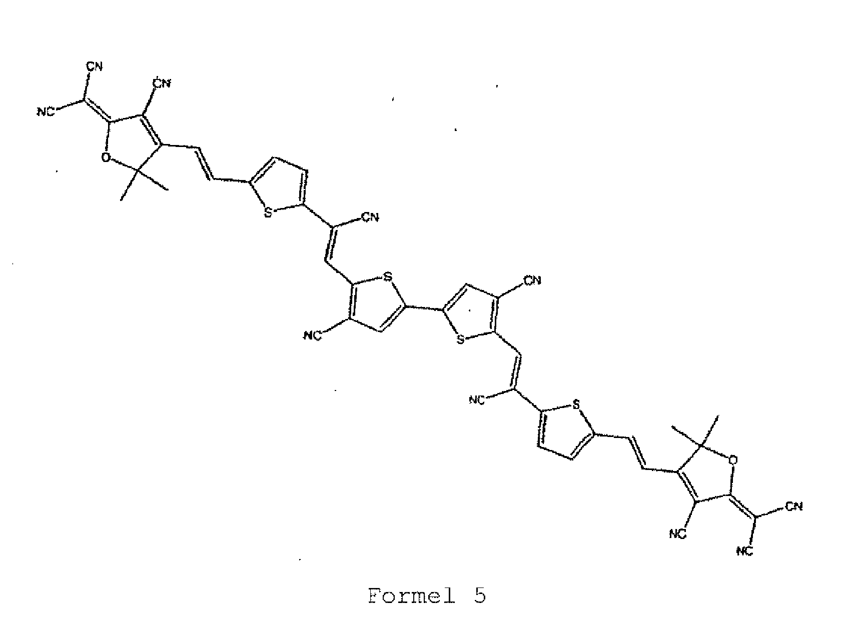

10. Transistor, **dadurch gekennzeichnet, dass** er wenigstens eine Schicht umfasst, die wenigstens ein Molekül der folgenden Formel (I) umfasst:
(GEA)ₙ-R-(DCDHF)ₘ Formel (I)
in der:
GEA eine elektronenanziehende Gruppe bezeichnet,
- n eine positive ganze Zahl zwischen 0 und 10, einschließlich, ist,
- R eine Gruppe mit konjugierten pi-Bindungen ist, die wenigstens einen aromatischen Ring mit 4 bis 6 Kettengliedern, einschließlich, umfasst, wobei dieser aromatische Ring gegebenenfalls wenigstens ein Heteroatom, ausgewählt aus N, 0, P, S, Si und Ge, umfasst und gegebenenfalls substituiert ist,
- DCDHF eine 2-Dicyanomethylen-3-cyano-2,5-dihydrofuran-Gruppe der folgenden Formel (II) darstellt: 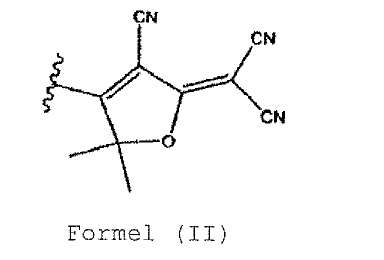
- m eine ganze Zahl zwischen 2 und 10, einschließlich, ist.

## Claims

1. Method of fabricating a transistor, **characterised in that** it includes a step using a molecule having the following formula (I):
(GEA)ₙ-R-(DCDHF)ₘ Formula (I)
in which:
- GEA designates an electron-withdrawing group,
- n is a positive integer between 0 and 10 inclusive,
- R is a group with conjugated pi bonds including at least one aromatic ring having 4 to 6 links inclusive, this aromatic ring optionally including at least one heteroatom chosen from N, 0, P, S, Si and Ge, and optionally being substituted,
- DCDHF represents a 2-dicyanomethylene-3-cyano-2,5-dihydrofuran group with the following formula (II): 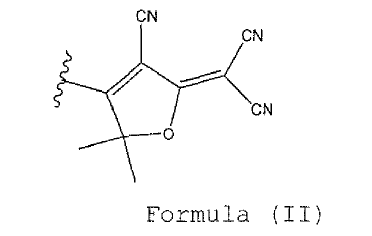
- m is an integer between 2 and 10 inclusive.

2. Method of fabricating a transistor, **characterised in that** it includes a step of depositing on at least one surface of a substrate a solution including at least one molecule having the following formula (I):
(GEA)ₙ-R-(DCDHF)ₘ Formula (I)
in which:
- GEA designates an electron-withdrawing group,
- n is a positive integer between 0 and 10 inclusive,
- R is a group with conjugated pi bonds including at least one aromatic ring having 4 to 6 links inclusive, this aromatic ring optionally including at least one heteroatom chosen from N, 0, P, S, Si and Ge, and optionally being substituted,
- DCDHF represents a 2-dicyanomethylene-3-cyano-2,5-dihydrofuran group with the following formula (II): 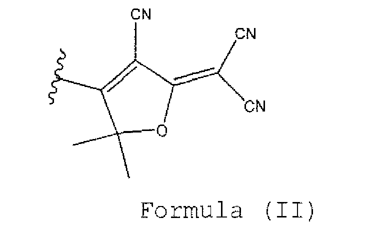
- m is an integer between 2 and 10 inclusive.

3. Method according to claim 1 or 2 of fabricating a transistor, **characterised in that**, in the molecule having the formula (I), the at least one aromatic ring of the group R is an aromatic ring substituted by unsaturated groups.

4. Method according to claim 1 or 2 of fabricating a transistor, **characterised in that** the compound having the formula (I) has at least one axis or plane of symmetry, advantageously a C₂ symmetry.

5. Method according to any one of the preceding claims **characterised in that**, in the compound having the formula (I), GEA is chosen from a fluorine atom, a cyano group, a fluorocarbon group, a malononitrile group, an anhydride group, an imide group, a nitro group, a quaternary ammonium group, an ester group, an amide group, a sulfonyl group and mixtures thereof.

6. Method according to any one of the preceding claims **characterised in that**, in the molecule having the formula (I), n is an integer between 0 and 4 inclusive.

7. Method according to any one of the preceding claims **characterised in that** the molecule having the formula (I) has one of the following formulas:

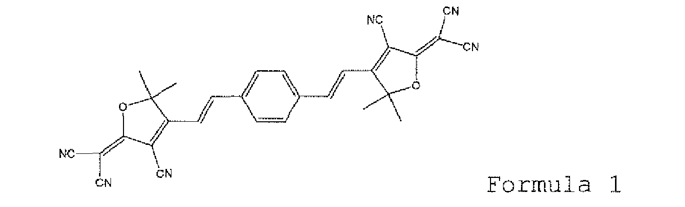

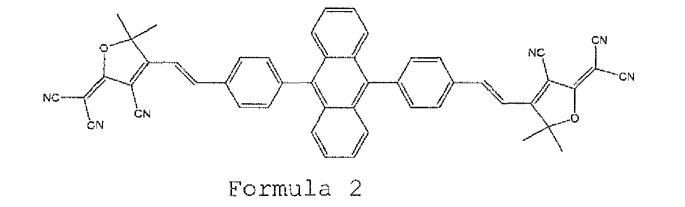

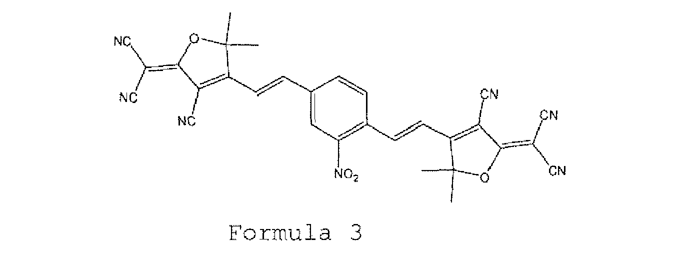

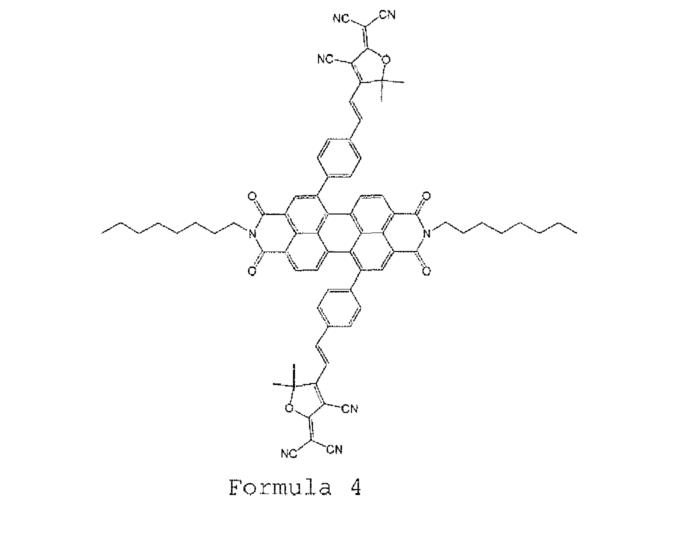

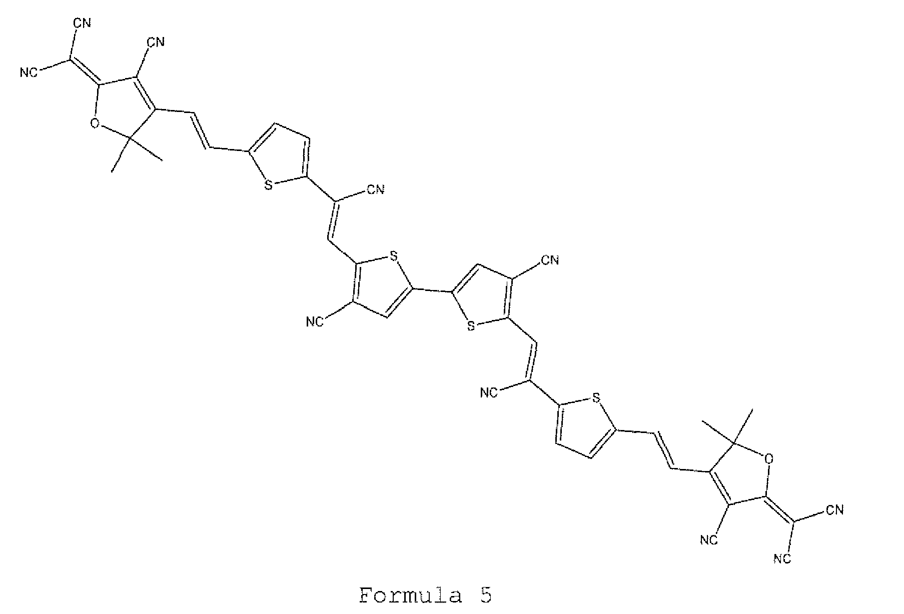

8. Molecule **characterised in that** it has the following formula (I):
(GEA)ₙ-R-(DCDHF)ₘ Formula (I)
in which:
- GEA designates an electron-withdrawing group,
- n is a positive integer between 0 and 10 inclusive,
- R is a group with conjugated pi bonds including at least one aromatic ring having 4 to 6 links inclusive, this aromatic ring optionally including at least one heteroatom chosen from N, 0, P, S, Si and Ge, and optionally being substituted,
- DCDHF represents a 2-dicyanomethylene-3-cyano-2,5-dihydrofuran group having the following formula (II): 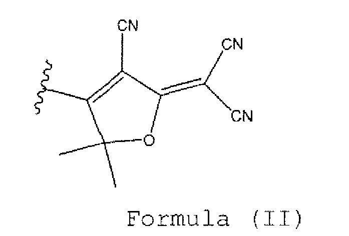
- m is an integer between 2 and 10 inclusive, provided that if n = 0, then the molecule has the following formula 1 or 2:

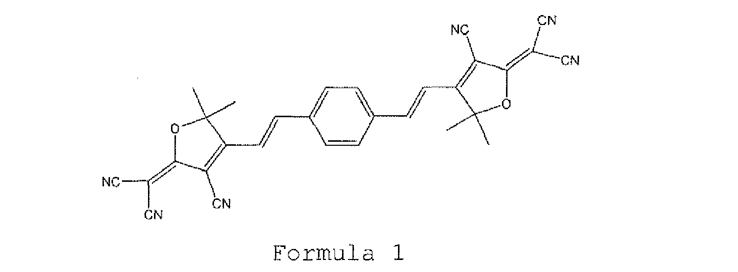

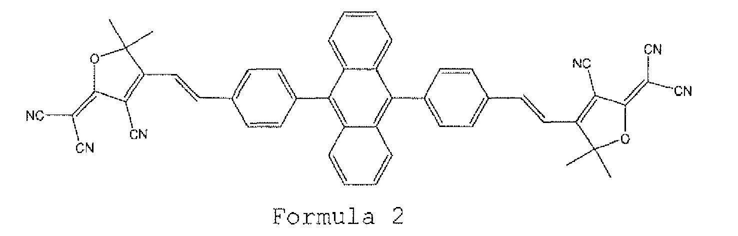

9. Molecule according to claim 8, **characterised in that** it is chosen from molecules with the following formulas 1 to 5:

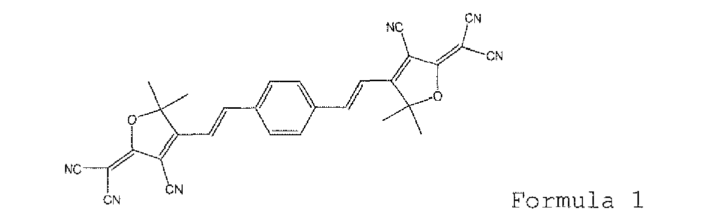

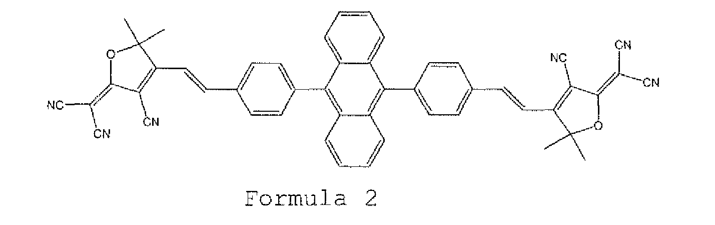

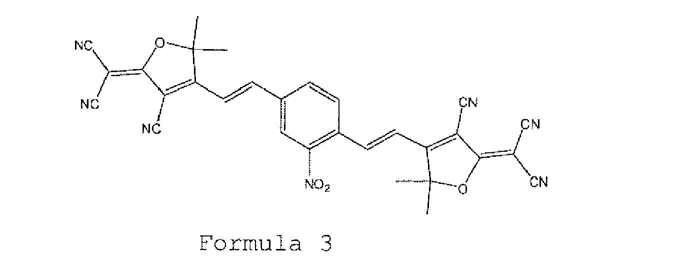

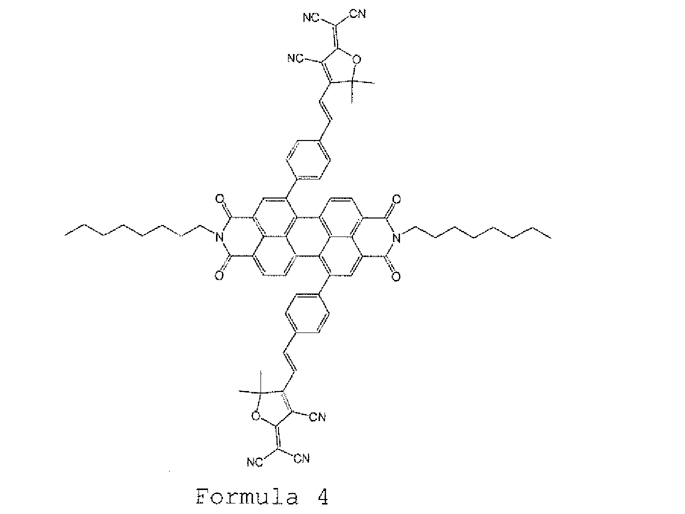

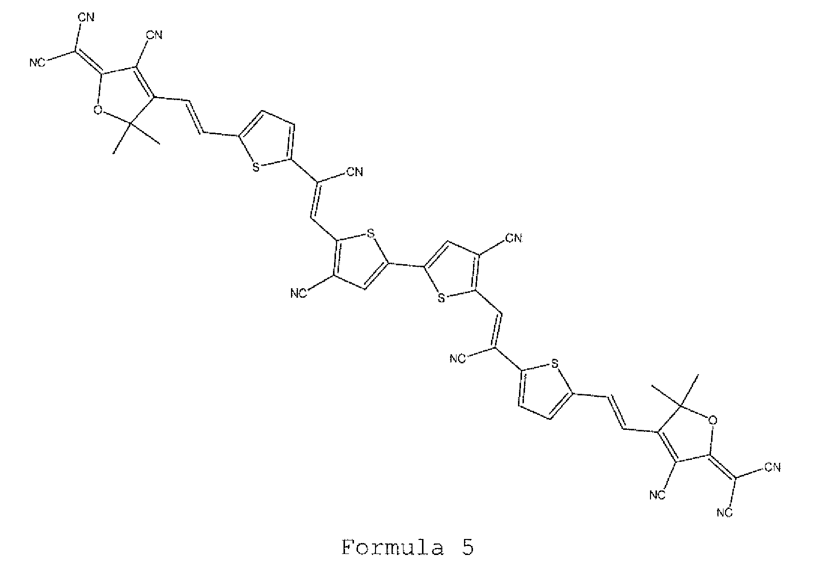

10. Transistor **characterised in that** it includes at least one layer including at least one molecule having the following formula (I):
(GEA)ₙ-R-(DCDHF)ₘ Formula (I)
in which :
- GEA designates an electron-withdrawing group,
- n is a positive integer between 0 and 10 inclusive,
- R is a group with conjugated pi bonds including at least one aromatic ring having 4 to 6 links inclusive, this aromatic ring optionally including at least one heteroatom chosen from N, 0, P, S, Si and Ge, and optionally being substituted,
- DCDHF represents a 2-dicyanomethylene-3-cyano-2,5-dihydrofuran group having the following formula (II): 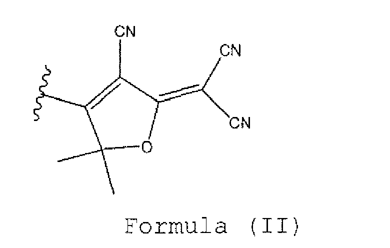
- m is an integer between 2 and 10 inclusive.
